# EUROPEAN PATENT SPECIFICATION

(11) **EP 3 223 736 B1**
(45) Date of publication and mention of the grant of the patent: **12.06.2019**
(21) Application number: 15718272.6
(22) Date of filing: 30.03.2015
(51) Int. Cl.: A61B 18/22, A61B 18/00

(54) **VARICOSE VEIN CLOSING LASER OPTIC FIBRE WINCH**
KRAMPFADERVERSCHLIESSENDE LASERLICHTLEITERWINDE
TREUIL DE FIBRE OPTIQUE POUR LASER DE FERMETURE DE VEINE VARIQUEUSE

(30) Priority: 25.11.2014 PL 41025514
(43) Date of publication of application: 04.10.2017
(73) Proprietor: ZORTRAX SPOLKA AKCYJNA, 10-409 Olsztyn (PL)
(72) Inventor: TOMASIAK, Rafal, 10-117 Olsztyn (PL); KLACZYNSKI, Robert, 82-200 Malbork (PL)
(74) Representative: Kancelaria Eupatent.pl Sp. z.o.o
(86) International application number: PCT/PL2015/000055
(87) International publication number: WO 2016/085358

(56) References cited:
- EP-A1- 1 574 176
- US-A1- 2004 199 151
- US-A1- 2010 152 538

## Description

The subject of the invention is a varicose vein closing laser optic fibre winch. The device may be used for ELVT method laser varicose vein removal treatment. It is used for withdrawing of the laser optic fibre from the patient's vein during the closing of the vessel using laser energy.

Currently, there is no device for aiding a physician in varicose vein laser removal treatments. A surgeon's assistant withdraws the optic fibre from the blood vessel with his fingers. He must ensure a uniform speed of withdrawal of the fibre, advantageously 1 mm/s, with a specific power of the laser beam. This method of fibre withdrawal cannot ensure a constant speed of vessel shrinking. Since the recommended speed of withdrawal is very low, for example removing 60 cm of fibre takes 10 minutes, the method of drawing the fibre with fingers is neither easy nor convenient for the operator.

A European patent application EP1574176 discloses a medical apparatus and method for efficacious thermal treatment of lumen, such as varicose veins, during laser surgery. It comprises a positioning device with two motors which are linked to two rollers, between which the optical fibre is engaged.

A US patent application US2004199151 discloses a system and method for controllably releasing laser radiation in percutaneous laser treatment. It does not disclose details of a mechanism for fiber advancement/withdrawal as to rollers driven by a motor.

A US patent application US2010152538 discloses a device that can be attached to an endoscopic device and when so attached is capable of protecting said endoscope from damage that may be caused by errant laser fibre. The device comprises a roller unit which can be configured to track passage of the fiber between a biasing member and a roller.

The object of the invention is a varicose vein closing laser optic fibre winch, as defined by the accompanying claims.

The optic fibre winch increases the accuracy of varicose vein closing through ensuring a uniform speed of laser optic fibre feeding speed. This makes the vessel uniformly shrunk over the entire length. Moreover, the use of the winch facilitates the surgery for the team, since it does not require manual, slow feeding of the laser optic fibre. Modular construction of the winch facilitates its washing and sterilisation. The laser fibre holder mechanism was designed in a manner which ensures that at any moment the laser fibre may be placed/released from the winch without needing to thread the end of the optic fibre through the winch.

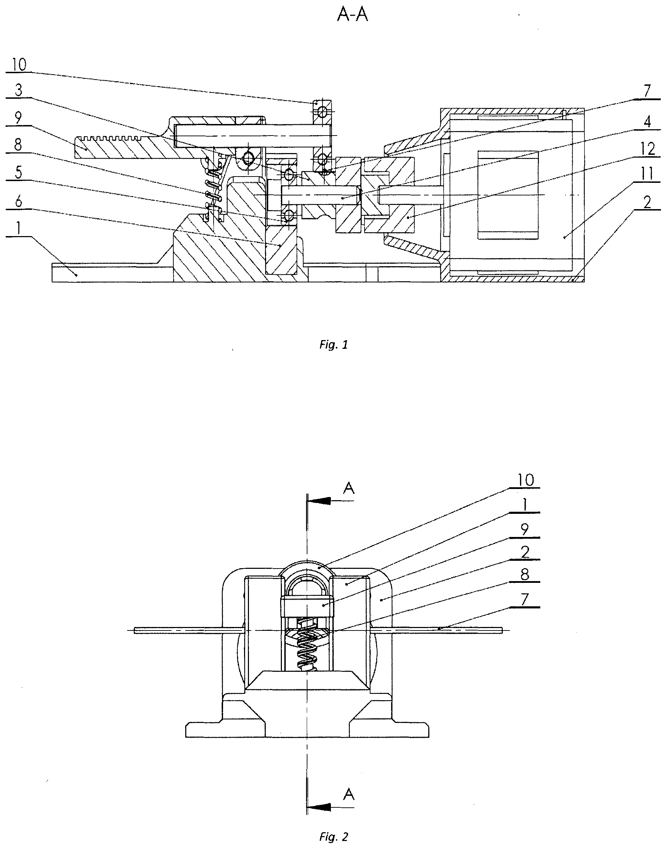

The subject of the invention will be presented in a sample implementation on figures, in which fig. 1 shows the laser optic fibre winch in a longitudinal section and fig. 2 shows the laser optic fibre winch in a side view.

The closing laser optic fibre winch is composed of a pulling section 1 and a drive section 2. In the pulling section body 1 there is a pulling mechanism composed of a drive roller 3 mounted on a shaft 4 rotating in a bearing 5 placed in a housing 6. The drive roller 3 has a toothed surface which ensures lack of slippage during movement of a laser optic fibre 7. A laser fibre 7 holder is adjacent to the drive roller 3, in the form of a spring 8 connected with a holder lever 9, on which a holder roller 10 is placed. In the driving section body 2 a motor 11 is located, advantageously a stepper motor, connected by a clutch 12 with a pulling part 1 of the roller 3.

### Operating principle

The laser optic fibre 7 is introduced into the limb vein. After it is introduced for a required length, it is placed in the winch, which is placed next to the limb near the vein entrance location of the fibre 7. The placement of the laser fibre 7 in the winch is performed by pressing the holder lever 9, which raises the holder roller 10 and enables the placement of the laser fibre 7 on the drive roller 3. Then a laser light is switched on. The laser power is absorbed by haemoglobin, which results in the thermal closing of the vessel lumen. Simultaneously with the vessel closing process, the withdrawing of the laser fibre 7 is started. For this purpose the motor 11 is started, which rotates the drive roller 3 through the clutch 12. The rotation of the drive roller 3 is changed to a linear motion of the laser fibre 7. The toothed surface of the drive roller 3 and the pressure of the holder roller ensures the movement of the laser fibre 7 without slippage. After the required length of the laser fibre 7 advancement is reached, the motor 1 is switched off, and the laser fibre 7 may be released by pressing again on the holder lever 9. In case of difficulties or any emergency situation, the laser fibre 7 may be at any time released from the winch, even during the operation of the motor 11.

After the treatment is finished it is possible to divide the winch manually into two parts, which facilitates the device's sterilisation. It consists of manually separating both parts without having to undo bolts or screws. The pulling section 1 of the winch, that is the one which comes into contact with the patient's blood contains moving greaseless elements, which enables immersing it in a disinfecting agent and then sterilising.

## Claims

1. A varicose vein closing laser optic fibre winch, comprising a pulling section (1) having a pulling mechanism comprising a drive roller (3) mounted on a shaft (4) rotating in a bearing (5) placed in a housing (6), said pulling mechanism furthermore comprising a laser fibre (7) holder adjacent to said drive roller (3), said laser fibre (7) holder comprising a spring (8) and a holder lever (9) connected thereto and having a holder roller (10), said winch furthermore comprising a drive section (2) separable from said pulling section (1) and containing a motor (11) connected by a clutch (12) with said drive roller (3) of said pulling mechanism.

2. The varicose vein closing laser optic fibre winch in accordance with claim 1, wherein said motor (11) is a stepper motor.

3. The varicose vein closing laser optic fibre winch in accordance with claim 1 or 2, wherein said drive roller (3) has a toothed surface which ensures movement of the laser fibre (7) without slippage.

## Patentansprüche

1. Laserglasfaserwinde zum Verschließen einer Krampfader, umfassend einen Zugabschnitt (1), der einen Zugmechanismus aufweist, der eine Antriebsrolle (3) umfasst, die an einer Welle (4) angebracht ist, die sich in einem Lager (5) dreht, das in einem Gehäuse (6) platziert ist, wobei der Zugmechanismus ferner einen Halter einer Laserfaser (7) benachbart zu der Antriebsrolle (3) umfasst, wobei der Halter einer Laserfaser (7) eine Feder (8) und einen damit verbundenen Haltehebel (9) umfasst und eine Halterolle (10) aufweist, wobei die Winde ferner einen Antriebsabschnitt (2) umfasst, der von dem Zugabschnitt (1) trennbar ist und einen Motor (11) enthält, der durch eine Kupplung (12) mit der Antriebsrolle (3) des Zugmechanismus verbunden ist.

2. Laserglasfaserwinde zum Verschließen einer Krampfader nach Anspruch 1, wobei der Motor (11) ein Schrittmotor ist.

3. Laserglasfaserwinde zum Verschließen einer Krampfader nach Anspruch 1 oder 2, wobei die Antriebsrolle (3) eine gezahnte Oberfläche aufweist, die Bewegung der Laserfaser (7) ohne Abrutschen sicherstellt.

## Revendications

1. Treuil de fibre optique pour laser de fermeture de veine variqueuse, comprenant une section de traction (1) possédant un mécanisme de traction comprenant un rouleau d'entraînement (3) monté sur un arbre (4) tournant dans un palier (5) placé dans un boîtier (6), ledit mécanisme de traction comprenant en outre un support de fibre laser (7) adjacent audit rouleau d'entraînement (3), ledit support de fibre laser (7) comprenant un ressort (8) et un levier de support (9) relié à celui-ci et possédant un rouleau de support (10), ledit treuil comprenant en outre une section d'entraînement (2) pouvant être séparée de ladite section de traction (1) et contenant un moteur (11) relié par un embrayage (12) au dit rouleau d'entraînement (3) dudit mécanisme de traction.

2. Treuil à fibre optique pour laser de fermeture de veine variqueuse selon la revendication 1, ledit moteur (11) étant un moteur pas à pas.

3. Treuil de fibre optique pour laser de fermeture de veine variqueuse selon la revendication 1 ou 2, ledit rouleau d'entraînement (3) possédant une surface dentée qui assure le déplacement de la fibre laser (7) sans glissement.
